# EUROPÄISCHE PATENTANMELDUNG

(11) **EP 3 400 877 A1**
(43) Veröffentlichungstag der Anmeldung: **14.11.2018**
(21) Anmeldenummer: 18152125.3
(22) Anmeldetag: 17.01.2018
(51) Int. Cl.: A61B 6/03, A61B 6/00

(54) **VERFAHREN ZUR BILDGEBUNG EINES UNTERSUCHUNGSBEREICHS MIT ERSTEM KONTRASTMITTEL UND ZWEITEM KONTRASTMITTEL**

(30) Priorität: 10.05.2017 EP 17170450
(71) Anmelder: Siemens Healthcare GmbH, 91052 Erlangen (DE)
(72) Erfinder: FLOHR, Thomas, 91486 Uehlfeld (DE); HARTUNG, Andre, 91052 Erlangen (DE); SCHMIDT, Bernhard, 90766 Fürth (DE)

(57) **Zusammenfassung**

Die Erfindung betrifft ein Verfahren (S) zur Bildgebung eines Untersuchungsbereichs eines zu untersuchenden Objekts (39), wobei der Untersuchungsbereich ein erstes Kontrastmittel und ein vom ersten Kontrastmittel verschiedenes zweites Kontrastmittel aufweist, mit einem Computertomographiesystem (31) aufweisend die Schritte der Aufnahme (S1) und der Erzeugung (S2). Im Schritt der Aufnahme (S1) werden erste Projektionsmessdaten (P1) mit einem ersten Energiebereich (E1) und von den ersten Projektionsmessdaten (P1) verschiedenen zweiten Projektionsmessdaten (P2) mit einem von dem ersten Energiebereich (E1) verschiedenen zweiten Energiebereich (E2) in einem Untersuchungsbereich aufgenommen. Im Schritt der Erzeugung (S2) wird ein ersten Bildes (B1) auf Basis der ersten Projektionsmessdaten (P1) und der zweiten Projektionsmessdaten (P2) erzeugt und das erste Bild (B1) weist ferner nur isolierte erste Informationen (I1) des ersten Kontrastmittels, nur isolierte zweite Informationen (I2) des zweiten Kontrastmittels, oder isolierte erste Informationen (I1) des ersten Kontrastmittels gemeinsam mit isolierten zweiten Informationen (I2) des zweiten Kontrastmittels auf.

## Beschreibung

Die Erfindung betrifft ein Verfahren sowie eine Bilderzeugungseinrichtung, ein Computertomographiesystem, ein Computerprogrammprodukt und ein computerlesbares Medium zur Unterscheidung bzw. getrennten Darstellung von Informationen eines ersten Kontrastmittels und eines zweiten Kontrastmittels in einem ersten Bild.

In der Röntgenbildgebung, beispielsweise in der Computertomographie, können energieaufgelöste Bilddaten bzw. Bilder erzeugt werden kann. Dazu können zählende bzw. energieauflösende Röntgendetektoren oder/und mehrere Röntgenspektren verwendet werden. Energieauflösende Röntgendetektoren können aus einer Aufnahme mit einem einzigen Röntgenspektrum oder mehreren Röntgenspektren energieaufgelöste Schwächungsdaten in mehreren Energiebereichen bereitstellen. Die Energiebereiche können dabei die im Konvertermaterial deponierte Energie oder, beispielsweise nach einem Schritt einer Entfaltung des Energiedepositionsspektrums mittels einer Detektorantwortfunktion, die Energie der Röntgenphotonen darstellen. Das Untersuchungsvolumen kann unter Verwendung mehrerer Röntgenspektren, beispielsweise in Verbindung mit mindestens einem integrierenden Röntgendetektor, aufgenommen werden. Dabei können beispielsweise mindestens zwei Röntgenquellen-Detektor-Systeme oder mindestens zwei verschiedenen Röntgenspektren einer einzigen Röntgenquelle verwendet werden.

Die Röntgenstrahlung oder die Photonen können in direkt-konvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden.

Die Computertomographie ist ein bildgebendes Verfahren, welches vor allem zur medizinischen Diagnostik sowie zur Materialuntersuchung eingesetzt wird. Bei der Computertomographie rotieren zur Aufnahme räumlich dreidimensionaler Bilddaten eine Strahlungsquelle, beispielsweise eine Röntgenquelle, sowie eine mit diesem zusammen wirkende Detektorvorrichtung um ein zu untersuchendes Objekt. Während der Rotationsbewegung werden innerhalb eines Winkelsektors Messdaten aufgenommen. Bei den Projektionsmessdaten handelt es sich um eine Vielzahl von Projektionen, welche Informationen über die Schwächung der Strahlung durch das Untersuchungsobjekt aus verschiedenen Projektionswinkeln enthalten. Aus diesen Projektionen lässt sich ein zweidimensionales Schnittbild oder ein dreidimensionales Volumenbild des Untersuchungsobjektes berechnen. Die Projektionsmessdaten werden auch als Rohdaten bezeichnet bzw. die Projektionsmessdaten können bereits vorverarbeitet sein, so dass beispielsweise detektorbedingte Intensitätsunterschiede der Schwächung reduziert sind. Aus diesen Projektionsmessdaten können dann Bilddaten rekonstruiert werden, beispielsweise mittels der sogenannten gefilterten Rückprojektion oder mittels eines iterativen Rekonstruktionsverfahrens, und damit ein Bild erzeugt werden.

Aus der Druckschrift WO 2016/146214 A1 ist ein System für eine transarterielle Chemoembolisation eines interessierenden Bereichs, umfassend einen Tumor, bekannt. Das System umfasst eine Injektionsvorrichtung, die so angeordnet ist, dass sie in den interessierenden Bereich erste Arzneimittel-eluierende Mikrosphärenperlen, die mindestens ein erstes Arzneimittel und ein erstes Kontrastmittel enthalten und in den interessierenden Bereich zweite Arzneimittel-eluierende Mikrokugelperlen einführen, die mindestens ein zweites Arzneimittel und ein zweites Kontrastmittel enthalten. Ein Abbildungssystem ist angeordnet, um einen ersten Bilddatensatz des interessierenden Bereichs mit mindestens einer ersten Röntgenstrahlungsenergie und einem zweiten Bilddatensatz des interessierenden Bereichs mit mindestens einer zweiten Röntgenstrahlungsenergie zu erhalten. Eine Konzentrationsbestimmungseinrichtung ist angeordnet, um eine erste Arzneimittelkonzentration aus dem ersten Bilddatensatz und eine zweite Arzneimittelkonzentration aus dem zweiten Bilddatensatz zu bestimmen.

Aus der Druckschrift US 2015/0221082 A1 ist ein Verfahren bekannt, welches das Bestimmen einer Permeabilitätsmetrik des interessierenden Gefäßgewebes auf der Grundlage einer ersten Zeitverstärkungskurve und einer zweiten Zeitverstärkungskurve umfasst, die einem ersten Kontrastmaterial und einem zweiten Kontrastmaterial entsprechen, welche durch das interessierende Gefäßgewebe fließen und ein Signal erzeugen, das dies anzeigt. Ein Rechensystem umfasst einen Zeitverstärkungskurvengenerator, der erste dynamisch kontrastverstärkte Bildgebungsdaten empfängt, die das interessierende Gefäßgewebe und ein erstes Kontrastmaterial mit schwach durchdringenden Partikeln darstellen, und das zweite dynamisch kontrastverstärkte Bildgebungsdaten empfängt, die das interessierende Gefäßgewebe und ein zweites Kontrastmaterial mit stark durchdringendem Partikel darstellen. Das Rechensystem erzeugt eine erste Zeitverstärkungskurve für das erste Kontrastmaterial und eine zweite Zeitverstärkungskurve für das zweite Kontrastmaterial. Eine Permeabilitätsmetrik-Bestimmungseinrichtung bestimmt eine Permeabilitätsmetrik für das interessierende Gefäßgewebe, indem eine effektive Differenz zwischen der ersten und der zweiten Zeitverstärkungskurve bestimmt wird.

Aus der Druckschrift US 2015/0038827 A1 ist eine medizinische Bilddiagnosevorrichtung, eine Abbildungseinheit, eine Bilderzeugungseinheit und eine Anzeigeeinheit bekannt. Die Bilderzeugungseinheit bildet ein Subjekt, in das Blutgefäßkontrastverstärkungspartikel und kranken Gewebekontrastverstärkungspartikeln injiziert wurden ab. Die Blutgefäßkontrastverstärkungspartikel haben die erste Partikelgröße, die größer ist als die Lücke der vaskulären Endothelzellen unter dem EPR-Effekt. Die erkrankten Gewebekontrastverstärkungspartikel haben die zweite Partikelgröße kleiner als die Lücke. Die Bilderzeugungseinheit erzeugt ein medizinisches Bild, das mit einem Abbildungsbereich des Subjekts verbunden ist, basierend auf Ausgangsdaten von der Abbildungseinheit. Die Anzeigeeinheit zeigt das medizinische Bild an.

Der Erfindung liegt das Problem zugrunde, dass bisher keine Möglichkeit zur Quantifizierung gleichzeitig im Körper vorhandener Kontrastmittel, beispielsweise in Verbindung mit Nanopartikeln oder Microspheres, in einer Aufnahme bzw. einem Bild besteht. Dadurch kann beispielsweise bei vorangegangenen Interventionen kein erstes Kontrastmittel verwendet werden, da das zweite Kontrastmittel vom ersten Kontrastmittel im Bild nicht unterschieden werden kann.

Es ist Aufgabe der Erfindung, ein Verfahren, eine Bilderzeugungseinrichtung, ein Computertomographiesystem, ein Computerprogrammprodukt und ein computerlesbares Medium anzugeben, welche eine Unterscheidung bzw. eine getrennten Darstellung von Informationen eines ersten Kontrastmittels und eines zweiten Kontrastmittels in einem ersten Bild ermöglichen.

Die Aufgabe wird erfindungsgemäß gelöst durch ein Verfahren nach Anspruch 1, eine Bilderzeugungseinrichtung nach Anspruch 20, ein Computertomographiesystem nach Anspruch 21, ein Computerprogrammprodukt nach Anspruch 22 und ein computerlesbares Medium nach Anspruch 23.

Die Erfindung betrifft ein Verfahren zur Bildgebung eines Untersuchungsbereichs eines zu untersuchenden Objekts, wobei der Untersuchungsbereich ein erstes Kontrastmittel und ein vom ersten Kontrastmittel verschiedenes zweites Kontrastmittel aufweist, mit einem Computertomographiesystem aufweisend die Schritte der Aufnahme von ersten Projektionsmessdaten und zweiten Projektionsmessdaten und der Erzeugung eines ersten Bildes aus den ersten Projektionsmessdaten und den zweiten Projektionsmessdaten. Die Aufnahme umfasst die Aufnahme von den ersten Projektionsmessdaten mit einem ersten Energiebereich und den von den ersten Projektionsmessdaten verschiedenen zweiten Projektionsmessdaten mit einem von dem ersten Energiebereich verschiedenen zweiten Energiebereich in einem Untersuchungsbereich. Die Erzeugung umfasst die Erzeugung des ersten Bildes auf Basis der ersten Projektionsmessdaten und auf Basis der zweiten Projektionsmessdaten. Die Erzeugung des ersten Bildes liefert ein erstes Bild aufweisend nur isolierte erste Informationen des ersten Kontrastmittels, nur isolierte zweite Informationen des zweiten Kontrastmittels, oder isolierte erste Informationen des ersten Kontrastmittels gemeinsam mit isolierten zweiten Informationen des zweiten Kontrastmittels. Das erste Bild kann beispielsweise zusätzlich ein Schwächungsbild des Untersuchungsbereichs umfassen. Die Schwächung durch das erste Kontrastmittel oder/und das zweite Kontrastmittel kann dabei unberücksichtigt sein bzw. durch die isolierte erste Information bzw. die isolierte zweite Information im ersten Bild dargestellt sein. Das Schwächungsbild kann beispielsweise zur anatomischen Orientierung dienen. Das erste Bild kann beispielsweise zusätzlich eine Markierung einer interessierenden Region aufweisen, in welcher eine Therapie mittels der therapeutisch wirksamen Funktionalisierung des ersten Kontrastmittels durchgeführt wird. Die isolierten ersten Informationen bzw. isolierten zweiten Informationen können im ersten Bild, beispielsweise als Graustufen oder farbige Schattierungen, oder als Zahlenwert angezeigt werden.

Die ersten Projektionsmessdaten und die zweiten Projektionsmessdaten werden bevorzugt mittels eines Computertomographiesystems (CT) aufgenommen. Die Aufnahme kann eine sequentielle Aufnahme oder eine Spiralaufnahme des Untersuchungsbereichs umfassen. Die ersten Projektionsmessdaten und die zweiten Projektionsmessdaten geben die Schwächung der Röntgenstrahlung durch das Objekt an. Die ersten Projektionsmessdaten und die zweiten Projektionsmessdaten geben im Wesentlichen ein Maß für die durch den Untersuchungsbereich gestrahlten Röntgenstrahlen an. Die ersten Projektionsmessdaten und die zweiten Projektionsmessdaten können vorverarbeitet sein, so dass beispielsweise Korrekturen der Detektorantwort berücksichtigt werden können. Die ersten Projektionsmessdaten und die zweiten Projektionsmessdaten umfassen bevorzugt Aufnahmen des gleichen Untersuchungsbereichs. Die Aufnahme kann insbesondere die Aufnahme von den ersten Projektionsmessdaten und von den zweiten Projektionsmessdaten des im Wesentlichen identischen Untersuchungsbereichs umfassen. Die Aufnahme der ersten Projektionsmessdaten oder die Aufnahme der zweiten Projektionsmessdaten können zusätzlich einen Bereich um den Untersuchungsbereich umfassen. Die ersten Projektionsmessdaten und die zweiten Projektionsmessdaten können bevorzugt zeitgleich aufgenommen werden. Die ersten Projektionsmessdaten und die zweiten Projektionsmessdaten können zeitlich versetzt aufgenommen werden.

Die ersten Projektionsmessdaten und die zweiten Projektionsmessdaten können für einen Schritt der Rekonstruktion verwendet werden. Die Rekonstruktion kann beispielsweise eine gefilterte Rückprojektion oder/und eine iterative Rekonstruktion umfassen. Das erste Bild kann entweder eine aus erster Information oder zweiter Information, oder erste Information und zweite Information gemeinsam, und dabei unterscheidbar, darstellen. Das erste Bild umfasst insbesondere den vollständigen Untersuchungsbereich.

Zur Visualisierung von Gefäßen oder zur Darstellung der Gewebeperfusion kann im Rahmen von CT-Untersuchungen ein zweites, beispielsweise jodhaltiges, Kontrastmittel, in der Regel intravenös, verabreicht werden. Auf Grund seiner hohen Ordnungszahl führt dieses zu einem guten Kontrast in CT-Bildern. Der Kontrast kann insbesondere gegenüber umliegendem Weichgewebe erhöht werden, welches im Wesentlichen kein erstes Kontrastmittel oder kein zweites Kontrastmittel aufweist. Angereicherte Strukturen, Läsionen oder Gefäße können so besser voneinander abgegrenzt werden. Im Rahmen von interventionellen Eingriffen kann ebenso Kontrastmittel verabreicht werden, wobei beispielsweise nur die Darstellung von Gefäßen im Vordergrund stehen kann. Der Untersuchungsbereich umfasst insbesondere einen, beispielsweise anatomisch oder physiologisch, relevanten Bereich des Untersuchungsobjekts für die Untersuchungsart bzw. für die anschließende, beispielsweise auf dem ersten Bild basierende, Diagnose oder Erfolgskontrolle einer Behandlung.

Darüber hinaus können auch noch andere Methoden oder Techniken zu einer Kontrastverstärkung in CT-Bildern führen. Im Rahmen von Studien konnte gezeigt werden, dass Nanopartikel, falls sie in ausreichender Konzentration vorliegen, mittels einer CT-Aufnahme dargestellt werden können. Zum Beispiel können sich auf Gold-basierte Nanopartikel in Plaques von Gefäßen in einer Konzentration anreichern, sodass ein Nachweis mit einer klinischen CT-Aufnahme möglich ist. Ein anderer Bereich, bei dem röntgensensitive Substanzen in den Körper gebracht werden, um eine Kontrastverstärkung zu erzielen, kann im Rahmen von Krebstherapien erfolgen. Bei einigen Methoden oder Techniken kann ein Therapeutikum, beispielsweise radioaktive Microspheres oder TARE, intra-arteriell im Rahmen einer Intervention direkt in den Bereich des Tumors eingebracht werden. Um die Verteilung des Therapeutikums, die Konzentration und auch das Verbleiben über die Zeit besser beurteilen zu können, kann dieses Therapeutikum mit einem Element hoher Ordnungszahl, beispielsweise Holmium, Eisen oder andere, oder einer Verbindung aufweisend ein Atom hoher Ordnungszahl vorab markiert werden. So kann die entsprechende Verteilung des Therapeutikums im CT-Bild bzw. ersten Bild sichtbar gemacht werden.

Das Verfahren kann ferner die Schritte der Steuerung von Injektionsvorrichtungen des ersten Kontrastmittels und des zweiten Kontrastmittels umfassen, wobei jede Steuerungseinheit ein insbesondere patientenspezifisches Injektionsprotokoll verwendet. Die Recheneinheit des Computertomographiesystems kann Parameter, beispielsweise einer Menge des ersten Kontrastmittels oder des zweiten Kontrastmittels oder des Injektionszeitpunkts, der Injektionsprotokolle bereitstellen oder/und empfangen.

Sowohl das erste Kontrastmittel, also auch das zweite Kontrastmittel, beispielsweise markierte Microspheres oder Nanopartikel, führen zu erhöhter Röntgenabsorption und damit zu höheren CT-Werten (in Hounsfield-Einheiten, HU) im ersten Bild bzw. in den CT-Bildern. Das erfindungsgemäße Verfahren ermöglicht, dass das erste Kontrastmittel bzw. das zweite Kontrastmittel für sich alleine dargestellt werden kann und quantifiziert werden kann. Sobald jedoch Methoden kombiniert werden, beispielsweise der Nachweis der Perfusion eines Tumors durch intravenös verabreichtes, jodhaltiges zweites Kontrastmittel bei gleichzeitiger Präsenz von beispielsweise holmiumhaltigen Microspheres als erstes Kontrastmittel aus einer vorausgegangenen Intervention, besteht bisher keine Möglichkeit zur Unterscheidung und Quantifizierung des ersten Kontrastmittels und des zweiten Kontrastmittels, weil im CT-Bild die einzelnen Substanzen allein aufgrund ihres CT-Wertes nicht voneinander getrennt werden können.

Die Erfinder schlagen vor, für die Unterscheidung von einem, beispielsweise arteriell oder intravenös verabreichten, zweiten Kontrastmittel von einem ersten Kontrastmittel, beispielsweise aufweisend Microspheres oder Nanopartikeln, eine spektrale Methode der Computertomographie zu verwenden. Abhängig von der Wahl des ersten Kontrastmittels und des zweiten Kontrastmittels kann die Datenaufnahme mit Dual-Energy-Techniken, bei welchen sich das röntgensensitive Material der Microspheres oder Nanopartikeln wie "dichtes" Wasser verhalten würde, oder mit Multienergietechniken erfolgen. Beispielsweise können mehrere Aufnahmen im Dual-Energy-Modus oder durch den Einsatz zählender Detektoren, welche die gleichzeitige Aufnahme mehrerer Energiebereiche ermöglichen, aufgenommen werden. Insbesondere vorteilhaft kann die Aufnahme mit zählenden Detektoren sein, es kann eine perfekte Übereinstimmung von mehreren Bildern, beispielsweise dem ersten Bild, dem zweiten Bild, dem dritten Bild, anatomisches CT-Bild, Kontrastmittelbild oder Nanopartikelbild, aufweisen, insbesondere durch gleichzeitige Aufnahme der ersten Projektionsmessdaten und der zweiten Projektionsmessdaten. Durch Anwendung von einer K-Kanten-Methode oder einer allgemeinen Methoden zur Multimaterialseparation auf die ersten Projektionsmessdaten und die zweiten Projektionsmessdaten kann vorteilhaft, trotz der Anwesenheit des ersten Kontrastmittels und des zweiten Kontrastmittels, das erste Kontrastmittel vom zweiten Kontrastmittel separiert werden und das erste Kontrastmittel und das zweite Kontrastmittel quantifizieren werden. Durch das zweite Kontrastmittel können angereicherte Strukturen, Läsionen oder Gefäße besser voneinander abgegrenzt werden, insbesondere auch bei Vorhandensein eines ersten Kontrastmittels im Untersuchungsbereich. Vorteilhaft kann eine Wartezeit zum Abbau eines zuvor verabreichten, insbesondere ersten, Kontrastmittels vermieden werden.

Die Aufnahme umfasst die Aufnahme von mindestens den ersten Projektionsmessdaten mit einem ersten Energiebereich und den von den ersten Projektionsmessdaten verschiedenen zweiten Projektionsmessdaten mit einem von dem ersten Energiebereich verschiedenen zweiten Energiebereich in einem Untersuchungsbereich. Es können darüber hinaus weitere Projektionsmessdaten insbesondere mit weiteren Energiebereichen aufgenommen werden. Die ersten, zweiten und ggf. weiteren Energiebereiche sind zumindest teilweise verschieden ausgebildet. Beispielsweise können mindestens zwei verschiedene Röntgenspektren als mindestens zwei verschiedene Energiebereiche verwendet werden. Beispielsweise können mindestens zwei verschiedene Detektorenergiebereiche als mindestens zwei verschiedene Energiebereiche verwendet werden. Es können mindestens zwei Projektionsmessdaten, insbesondere mindestens zwei Projektionsmessdatensätze, aufgenommen werden. Die Anzahl der Energiebereiche bzw. Projektionsmessdaten(-sätze) kann insbesondere im Bereich von 2 bis 10, bevorzugt im Bereich von 2 bis 4 liegen. Besonders bevorzugt kann die Anzahl der Energiebereiche bzw. Projektionsmessdaten(-sätze) 2, 3 oder 4 betragen. Die Anzahl der Energiebereiche bzw. Projektionsmessdaten(-sätze) kann beispielsweise im Bereich von 2 bis 8 oder im Bereich von 2 bis 6 liegen. Die Anzahl der Energiebereiche bzw. Projektionsmessdaten(-sätze) kann insbesondere ganzzahlig sein. Vorteilhaft kann eine Verwendung von mindestens zwei Energiebereichen die spektrale Auflösung der Aufnahme erhöhen. Besonders vorteilhaft kann die spektrale Auflösung der Aufnahme durch die Verwendung von mehr als zwei Energiebereichen erhöht werden.

Die Erfinder schlagen insbesondere vor ein während einer Intervention verabreichtes, an Mikrokugeln (engl.: microspheres) oder Nanopartikel gebundene Kontrastsubstanz als erstes Kontrastmittel von einem während oder nach der Intervention intraarteriell oder intravenös verabreichten zweiten Kontrastmittel durch Aufnahme von CT-Datensätzen bei mindestens zwei unterschiedlichen Röntgenenergien zu trennen.

Die Erfinder haben erkannt, dass in einer Single-Energy-CT Untersuchung das erste Kontrastmittel und das zweite Kontrastmittel nicht trennbar sind. Mittels spektraler Computertomographie können beispielsweise zwei Bilder berechnet werden, nämlich ein erstes Bild, das nur das erste Kontrastmittel enthält und das man zur Ermittlung der Reichweite der Therapie verwenden kann, und ein zweites Bild, das nur das zweite Kontrastmittel enthält, und das man, weil es ein Maß für die lokale Durchblutung ist, zur Ermittlung der Wirksamkeit der Therapie verwenden kann. Die spektrale Computertomographie kann einen Dual-Energy-Ansatz mit zwei verschiedenen Röntgenspektren oder einen spektral auflösenden bzw. (photonen-)zählenden Röntgendetektor umfassen.

Vorteilhaft kann eine Trennung und eine Quantifizierung eines beispielsweise radioaktiven, deponierten Therapeutikums, beispielsweise im Rahmen einer Intervention (TARE) in Form von Microspheres, und eines beispielsweise währenddessen oder im Anschluss an die Intervention intravenös verabreichten zweiten Kontrastmittels basierend auf Grund von spektraler Informationen erhalten werden. Vorteilhaft können die Trennungen und die Quantifizierung von Nanopartikeln, beispielsweise verabreicht im Rahmen einer Intervention als erstes Kontrastmittel, und intravenös verabreichtem zweiten Kontrastmittel auf Grund von spektralen Informationen erhalten werden. Vorteilhaft kann die Darstellung der isolierten ersten Information und der isolierten zweiten Information nicht durch Bewegung zwischen CT-Untersuchungen beeinflusst sein.

Gemäß einem Aspekt der Erfindung weist das erste Kontrastmittel eine therapeutische wirksame Funktionalisierung auf. An das erste Kontrastmittel kann eine therapeutisch wirksame Substanz gekoppelt sein. Das erste Kontrastmittel kann eine therapeutische wirksame Funktionalisierung aufweisen. Das erste Kontrastmittel kann in Form von Nanopartikeln, Microspheres oder Ähnlichem vorliegen und entweder chemisch oder durch Strahlung therapeutisch wirksam sein. Das erste Kontrastmittel kann bei der Intervention, beispielsweise in die Leber, eingebracht werden und verbleibt dort. Vorteilhaft kann das erste Kontrastmittel gleichzeitig eine therapeutisch wirksame Funktionalisierung und eine Kontrastmittelsubstanz aufweisen.

Gemäß einem Aspekt der Erfindung ist das zweite Kontrastmittel für eine Blutflusseigenschaft indikativ. Das zweite Kontrastmittel kann eine Blutflusseigenschaft in einem Bereich oder Volumen des Untersuchungsbereichs anzeigen. Das zweite Kontrastmittel kann bevorzugt ein bekanntes Kontrastmittel sein, beispielsweise aufweisend Jod, Gadolinium oder andere, mit dem die Durchblutung untersucht bzw. dargestellt werden kann. Das zweite Kontrastmittel kann gleichzeitig oder in einer Folgeuntersuchung nach der Intervention gegeben werden. Vorteilhaft kann der Behandlungserfolg der therapeutisch wirksamen Funktionalisierung dargestellt bzw. festgestellt werden. Es kann ferner eine globale Blutflusseigenschaft angezeigt werden.

Gemäß einem Aspekt der Erfindung weist das erste Bild eine lokale Information über eine lokale Verteilung des ersten Kontrastmittels oder/und zweiten Kontrastmittels auf. Die Verteilung im ersten Bild kann angeben, ob das erste Kontrastmittel oder/und das zweite Kontrastmittel in lokalen Volumen des Untersuchungsbereichs vorhanden sind oder nicht. Durch Aufnahme von den ersten Projektionsmessdaten und den zweiten Projektionsmessdaten, auch bezeichenbar als CT-Datensätzen bei mindestens zwei unterschiedlichen Röntgenenergien, kann die lokale Verteilung des beispielsweise während einer Intervention verabreichten, an Mikrokugeln oder Nanopartikel gebundenen ersten Kontrastmittels dargestellt und quantifiziert werden. Ferner kann die lokale Verteilung des beispielsweise während oder nach der Intervention intra-arteriell oder intravenös verabreichten zweiten Kontrastmittels dargestellt und quantifiziert werden. Vorteilhaft können das erste Kontrastmittel und das zweite Kontrastmittel sowie deren Menge bzw. lokale Verteilung getrennt voneinander dargestellt werden. Die lokale Verteilung oder die lokale Information kann beispielsweise durch Überlagerung über einer anatomischen Darstellung im ersten Bild, im zweiten Bild oder im dritten Bild dargestellt werden. Die lokale Information oder die lokale Verteilung kann beispielsweise farbig oder/und mit einer Textur bzw. Schattierung dargestellt sein. Es kann ferner ein globaler Parameter über die lokale Verteilung, beispielsweise als Summe der lokalen Informationen, angezeigt werden.

Gemäß einem Aspekt der Erfindung weist das erste Bild eine lokale Information über eine Menge des ersten Kontrastmittels oder/und des zweiten Kontrastmittels auf. Die Menge kann eine absolute Menge, eine Dichte oder eine Konzentration sein. Neben der Verteilung kann auch eine lokale Information über die Menge dargestellt werden. Die lokale Information kann beispielsweise für den gesamten Untersuchungsbereich, einen Teilbereich des Untersuchungsbereichs bis hin zu einem Voxel des ersten Bildes dargestellt werden. Vorteilhaft kann ein Maß für die Deposition des ersten Kontrastmittels, insbesondere mit therapeutisch wirksamer Funktionalisierung, im Untersuchungsbereich bzw. Behandlungsbereich bestimmt und dargestellt werden. Vorteilhaft kann ein Maß für die Deposition des zweiten Kontrastmittels, insbesondere zur Darstellung der Durchblutung, im Untersuchungsbereich bzw. Behandlungsbereich bestimmt und dargestellt werden. Es kann ferner ein globaler Parameter über die Menge, beispielsweise als Summe der lokalen Informationen, angezeigt werden.

Gemäß einem Aspekt der Erfindung weist das erste Bild eine lokale Information über eine Menge oder Verteilung des ersten Kontrastmittels aufweisend eine therapeutische wirksame Funktionalisierung auf. Die Verteilung und lokale Dichte bzw. die lokale Information des ersten Kontrastmittels kann als Maß für die Verteilung und lokale Menge bzw. Dichte von bei der Intervention verabreichten und an die Mikrokugeln oder Nanopartikel gebundenen chemischen oder radioaktiven Substanzen, beispielsweise Transarterielle Chemoembolisation (TACE) oder Transarterielle Radioembolisation (TARE), und damit vorteilhaft als Maß für die Reichweite der Intervention dienen. Die erste Information bzw. die Verteilung und die lokale Dichte des während oder nach der Intervention intraarteriell oder intravenös verabreichten zweiten Kontrastmittels kann vorteilhaft als Maß für die lokale Durchblutung und damit für den Therapieerfolg dienen. Es kann ferner ein globaler Parameter über die Menge, beispielsweise als Summe der lokalen Informationen, angezeigt werden.

Gemäß einem Aspekt der Erfindung umfasst die therapeutische wirksame Funktionalisierung an das erste Kontrastmittel gebundene chemische oder/und radioaktive Substanzen bzw. eine chemische oder/und radioaktive Substanz. Das erste Kontrastmittel kann chemische Substanzen, beispielsweise zur Transateriellen Chemoembolisation (TACE), aufweisen. Das erste Kontrastmittel kann radioaktive Substanzen, beispielsweise zur Transateriellen Radioembolisation (TARE), aufweisen. Vorteilhaft kann das erste Kontrastmittel mehrere Funktionen wie therapeutisch wirksame Funktion und Kontrastverstärkung aufweisen.

Die Radioembolisation oder selektive interne Radio-Therapie (SIRT) kann beispielsweise für primäre und sekundäre Lebertumoren verwendet werden. Die Radioembolisation erfolgt durch Injektion des ersten Kontrastmittels über beispielsweise die Leberarterie. Das erste Kontrastmittel kann eine radioaktive Substanz, beispielsweise Yttrium-90, aufweisen. Durch eine in der Regel stärkere Durchblutung im Tumor kann eine besonders hohe Konzentration bzw. Dichte des ersten Kontrastmittels vorteilhaft erreicht werden. Das Tumorgewebe kann durch die radioaktive Substanz abgetötet werden, so dass der Tumor zumindest teilweise zurückgehen kann.

Gemäß einem Aspekt der Erfindung weist das erste Bild eine lokale Information über eine lokale Verteilung des ersten Kontrastmittels oder/und des zweiten Kontrastmittels in einem vorbestimmten Bereich auf. Der vorbestimmte Bereich kann ein Behandlungsbereich sein. Der vorbestimmte Bereich kann das mit dem ersten Kontrastmittel bzw. dessen therapeutisch wirksamer Funktionalisierung zu behandelnde Volumen im Untersuchungsbereich bezeichnen. Der vorbestimmte Bereich kann im ersten Bild markiert, beispielsweise durch eine Umrandung oder flächige Markierung, sein. Vorteilhaft kann durch visuellen Vergleich oder automatischen Vergleich des vorbestimmten Bereichs mit der lokalen Information über eine lokale Verteilung die Reichweite des ersten Kontrastmittels bzw. der Erfolg des Einbringens im vorbestimmten Bereich ermittelt werden.

Gemäß einem Aspekt der Erfindung weist das erste Bild eine lokale Information basierend auf dem zweiten Kontrastmittel über eine lokale Durchblutung in einem vorbestimmten Bereich auf. Die lokale Durchblutung in dem vorbestimmten Bereich kann eine Information über den Therapieerfolg liefern. Vorteilhaft kann durch visuellen Vergleich oder automatischen Vergleich des vorbestimmten Bereichs mit der lokale Information basierend auf dem zweiten Kontrastmittel über eine lokale Durchblutung der Therapieerfolg im vorbestimmten Bereich ermittelt werden. Beispielsweise kann durch die therapeutisch wirksame Funktionalisierung ein Tumor als vorbestimmter Bereich behandelt werden. Weist der vorbestimmte Bereich eine geringe oder keine Durchblutung auf, so kann auf einen Therapieerfolg geschlossen werden. Die Beurteilung des Therapieerfolgs kann ferner Biomarker oder eine Texturanalyse, beispielsweise der Verteilung des ersten Kontrastmittels oder des zweiten Kontrastmittels, umfassen.

Gemäß einem Aspekt der Erfindung wird ein zweites Bild mit isolierten zweiten Informationen des zweiten Kontrastmittels und entfernten isolierten ersten Informationen des ersten Kontrastmittels erzeugt. Zusätzlich zum ersten Bild kann ein zweites Bild erzeugt werden. Alternativ kann das zweite Bild dem ersten Bild entsprechen. Das zweite Bild kann nur isolierte zweite Informationen des zweiten Kontrastmittels aufweisen. Das erste Bild bzw. das zweite Bild kann derart mittels spektraler Techniken erzeugt werden, dass das erste Kontrastmittel durch spektrale Techniken entfernt ist und nur das zweite Kontrastmittel dargestellt wird. Die Entfernung des ersten Kontrastmittels kann beispielsweise mittels (Multi-)Materialzerlegung erreicht werden. Vorteilhaft kann die Überprüfung der Durchblutung mittels des zweiten Kontrastmittels in ähnlicher Qualität durchgeführt werden im Vergleich zu einer Untersuchung unter Abwesenheit des ersten Kontrastmittels.

Gemäß einem Aspekt der Erfindung wird ein zweites Bild mit isolierten ersten Informationen des ersten Kontrastmittels und entfernten isolierten zweiten Informationen des zweiten Kontrastmittels erzeugt. Das zweite Bild kann nur isolierte erste Informationen des ersten Kontrastmittels aufweisen. Das erste Bild bzw. das zweite Bild kann derart mittels spektraler Techniken erzeugt werden, dass das zweite Kontrastmittel durch spektrale Techniken entfernt ist und nur das erste Kontrastmittel dargestellt wird. Die Entfernung des zweiten Kontrastmittels kann beispielsweise mittels (Multi-)Materialzerlegung erreicht werden. Vorteilhaft kann die Überprüfung der Reichweite des ersten Kontrastmittels in ähnlicher Qualität durchgeführt werden im Vergleich zu einer Untersuchung unter Abwesenheit des zweiten Kontrastmittels.

Gemäß einem Aspekt der Erfindung wird ein drittes Bild mit entfernten isolierten ersten Informationen des ersten Kontrastmittels und mit entfernten isolierten zweiten Informationen des zweiten Kontrastmittels erzeugt. Aus dem dritten Bild können die ersten Informationen des ersten Kontrastmittels und die zweiten Informationen des zweiten Kontrastmittels durch spektrale Techniken entfernt sein. Vorteilhaft kann im dritten Bild ausschließlich anatomische Information ohne Einfluss von Kontrastmitteln dargestellt sein. Vorteilhaft kann im vorbestimmten Bereich eine genauere Diagnose, beispielsweise hinsichtlich der anatomischen Umgebung um einen mit dem ersten Kontrastmittel oder dem zweiten Kontrastmittel markierten Bereich, durchgeführt werden.

Gemäß einem Aspekt der Erfindung weist das erste Kontrastmittel eine an Mikrokugeln oder Nanopartikel gebundene Kontrastsubstanz auf. Vorteilhaft können die Mikrokugeln oder Nanopartikel als Träger für die Kontrastsubstanz dienen, so dass sie gemeinsam mit der therapeutisch wirksamen Funktionalisierung das erste Kontrastmittel bilden.

Gemäß einem Aspekt der Erfindung weist das erste Kontrastmittel mindestens eines der Elemente Holmium, Eisen, Gold, Wolfram oder Gadolinium auf. Die an das erste Kontrastmittel gebundene Kontrastsubstanz kann Holmium oder Eisen oder Gold oder Wolfram oder Gadolinium enthalten. Das erste Kontrastmittel kann als Kontrastsubstanz insbesondere ein Element mit einer Ordnungszahl größer als 20 aufweisen. Das erste Kontrastmittel kann als Kontrastsubstanz insbesondere ein Element mit einer Ordnungszahl kleiner als 80 aufweisen. Die Kontrastsubstanz erfüllt die Bedingung, dass sie in der für die Untersuchung oder Behandlung nötigen Dosierung nicht schädlich für das Untersuchungsobjekt ist. Vorteilhaft kann das erste Kontrastmittel von Knochen unterschieden werden.

Gemäß einem Aspekt der Erfindung weist das zweite Kontrastmittel mindestens eines der Elemente Jod oder Gadolinium auf. Als zweites Kontrastmittel kann Jod oder Gadolinium verwendet werden. Falls das erste Kontrastmittel Jod aufweist, so weist das zweite Kontrastmittel beispielsweise Gadolinium auf. Falls das erste Kontrastmittel Gadolinium aufweist, so weist das zweite Kontrastmittel beispielsweise Jod auf. Vorteilhaft kann das zweite Kontrastmittel ein bekanntes, zur Untersuchung der Durchblutung geeignetes Kontrastmittel sein, welches beispielsweise in der nötigen Dosis nicht toxisch ist. Vorteilhaft können das erste Kontrastmittel und das zweite Kontrastmittel durch ihre Verschiedenartigkeit mittels einer spektralen Technik unterschieden werden. Die Verschiedenartigkeit der beiden Kontrastmittel kann beispielsweise durch unterschiedliche Wirkungsquerschnitte bzw. Schwächungskoeffizienten hinsichtlich Photo- bzw. Compton-Effekt ausgebildet sein. Die Verschiedenartigkeit der beiden Kontrastmittel kann beispielsweise hinsichtlich der erhöhten Absorption bedingt durch eine K-Kante des ersten Kontrastmittels oder des zweiten Kontrastmittels ausgebildet sein. Die beiden Kontrastmittel können derart verschieden ausgebildet sein, dass sie mittels Methoden der spektralen Computertomographie unterscheidbar sind. Die beiden Kontrastmittel können insbesondere einen unterschiedlichen energieabhängigen Schwächungskoeffizienten aufweisen.
Gemäß einem Aspekt der Erfindung weist das erste Kontrastmittel oder das zweite Kontrastmittel ein Element auf, welches eine K-Kante im Bereich von 30 bis 100keV aufweist. Beispielsweise weisen Jod, Gadolinium und Gold eine K-Kante auf. Ferner sind Jod, Gadolinium und Gold als Kontrastmittel geeignet. Durch die K-Kante des ersten Kontrastmittels oder des zweiten Kontrastmittels kann mittels spektraler Technik, insbesondere einem zählenden bzw. direkt-konvertierenden Röntgendetektor, der erreichbare Kontrast im beispielsweise ersten Bild oder zweiten Bild vorteilhaft erhöht wird. Bevorzugt weist entweder das erste Kontrastmittel oder das zweite Kontrastmittel eine K-Kante im Bereich von 30 bis 100keV auf.

Der erste Energiebereich oder der zweite Energiebereich kann bevorzugt an die Photonenenergie, bei der der Energiewert der K-Kante erreicht bzw. überschritten ist, angepasst sein. Es kann zunächst für den ersten Energiebereich ein erstes Zwischenbild und für den zweiten Energiebereich ein zweites Zwischenbild erzeugt werden. Anschließend kann ein Differenzbild aus dem ersten Zwischenbild und dem zweiten Zwischenbild erzeugt werden. Dabei kann die unterschiedliche Abhängigkeit der Schwächungskoeffizienten von der Photonenenergie der Röntgenstrahlung des ersten Kontrastmittels und des zweiten Kontrastmittels vorteilhaft genutzt werden, wobei die Abhängigkeit besonders stark im Bereich der K-Kante ausgeprägt ist. Besonders vorteilhaft ist die Wahl des ersten Energiebereichs und des zweiten Energiebereichs, wenn jeweils einer der beiden Energiebereiche oberhalb bzw. unterhalb des Energiewerts der K-Kante ausgebildet ist. Das Differenzbild kann das erste Bild oder das zweite Bild sein. Das Differenzbild kann nur isolierte Informationen des Kontrastmittels, welches ein Element mit einer K-Kante aufweist, aufweisen.

Gemäß einem Aspekt der Erfindung umfasst der erste Energiebereich ein erstes Röntgenspektrum und der zweite Energiebereich ein zweites Röntgenspektrum. Der erste Energiebereich und der zweite Energiebereich können mittels zwei unterschiedlicher Röhrenspannungen oder Filterungen eingestellt werden. Beispielsweise kann ein Dual-Energy-fähiges oder/und Dual-Source-Computertomographiesystem verwendet werden. Beispielsweise kann kV-Switching verwendet werden. Die Aufnahmen der ersten Projektionsmessdaten und der zweiten Projektionsmessdaten können zeitlich oder/und räumlich versetzt aufgenommen werden, beispielsweise bedingt durch das Umschalten der Röhrenspannung bzw. Filterung oder der Anordnung der zwei Röntgenröhren-Detektorsysteme. Vorteilhaft können mit einfachen Mitteln, beispielsweise das Umschalten der Röhrenspannung bzw. Filterung, zwei voneinander verschiedene Energiebereiche für die Aufnahme bereitgestellt werden. Das erste Röntgenspektrum kann beispielsweise eine Röhrenspannung zwischen 70 kV und 100 kV aufweisen. Das zweite Röntgenspektrum kann beispielsweise eine Röhrenspannung zwischen 120 kV und 150 kV aufweisen. Der erste Energiebereich und der zweite Energiebereich können teilweise überlappend ausgebildet sein, insbesondere in einem Energieteilbereich unterhalb der maximalen Photonenenergie des ersten Röntgenspektrums, wobei die Röhrenspannung des zweiten Röntgenspektrums höher als die Röhrenspannung des ersten Röntgenspektrums ausgebildet ist.

Gemäß einem Aspekt der Erfindung umfasst der erste Energiebereich einen ersten Detektorenergiebereich und der zweite Energiebereich einen zweiten Detektorenergiebereich. Der erste Detektorenergiebereich und der zweite Detektorenergiebereich können bevorzugt in einem direkt-konvertierenden bzw. zählenden Röntgendetektor ausgebildet sein. Der erste Detektorenergiebereich bzw. der zweite Detektorenergiebereich kann durch vorbestimmte Schwellwerte festgelegt sein. Beispielsweise kann der erste Detektorenergiebereich bzw. der zweite Detektorenergiebereich durch einseitige Diskriminierung mittels eines Schwellwerts oder durch zweiseitige Diskriminierung, eine sogenannte Fensterdiskriminierung, mittels zwei verschiedener Schwellwerte festgelegt sein. Der erste Detektorenergiebereich und der zweite Detektorenergiebereich weisen voneinander getrennte Register zum Zählen der Ereignisse oberhalb des Schwellwertes bzw. zwischen den beiden Schwellwerten auf. Im Fall einer einseitigen Diskriminierung können der erste Detektorenergiebereich und der zweite Detektorenergiebereich zumindest teilweise überlappen. Im Fall einer zweiseitigen Diskriminierung können der erste Detektorenergiebereich und der zweite Detektorenergiebereich im Wesentlichen voneinander getrennt bzw. im Wesentlichen nichtüberlappend ausgebildet sein. Die im Wesentlichen getrennten Detektorenergiebereiche können im Rahmen der Genauigkeit der Diskriminierung der elektrischen Signale voneinander getrennt sein. Die Trennung bezieht sich dabei insbesondere auf die Unterscheidung unterschiedlicher elektrischer Signale basierend auf der unterschiedlichen deponierten Energie im Konvertermaterial. Hinsichtlich der Trennung von Photonenenergien kann die im Wesentliche Trennung der Detektorenergiebereiche beispielsweise aufgrund der Detektorantwortfunktion eine größere Ungenauigkeit gegenüber der Trennung rein basierend auf den unterschiedlichen elektrischen Signalen aufweisen. Vorteilhaft können die ersten Projektionsmessdaten und die zweiten Projektionsmessdaten gleichzeitig aufgenommen werden. Der Röntgendetektor kann bevorzugt 2, 3 oder 4 Detektorenergiebereiche, insbesondere pro Detektorelement oder (Sub-)pixel, aufweisen. Alternativ oder zusätzlich können beispielsweise benachbarte Detektorelement oder benachbarte (Sub-)Pixel verschiedene Detektorenergiebereiche aufweisen, wobei jedes Detektorelement oder jeder (Sub-)Pixel mindestens einen Detektorenergiebereich aufweist.

Gemäß einem Aspekt der Erfindung ist ein Schritt der Multimaterialzerlegung vom erfindungsgemäßen Verfahren umfasst. Die Multimaterialzerlegung kann rohdatenbasiert oder bilddatenbasiert erfolgen. Die (Multi-)Materialzerlegung kann beispielsweise die unterschiedlichen Wirkungsquerschnitte verschiedener Elemente für Photo- und Comptoneffekt ausnutzen. Es können zwei Basismaterialien gewählt werden. Es kann beispielsweise eine segmentierungsgestützte Materialquantifizierung mit mehreren Materialzerlegungen durchgeführt werden, wobei die ersten Projektionsmessdaten und die zweiten Projektionsmessdaten mittels spektraler Informationen segmentiert werden sowie Vorwissen und Heuristiken einfließen können. Dabei können die ersten Projektionsmessdaten und die zweiten Projektionsmessdaten in Fettgewebe, Gewebe, dichtes Gewebe, Knochen und Luft unterteilt werden. Die ersten Projektionsmessdaten und die zweiten Projektionsmessdaten können bevorzugt in zwei aus Fettgewebe, Gewebe, dichtes Gewebe, Knochen und Luft sowie in erstes Kontrastmittel und zweites Kontrastmittel unterteilt werden. Vorteilhaft kann das erste Kontrastmittel und das zweite Kontrastmittel von zwei aus Fettgewebe, Gewebe, dichtes Gewebe, Knochen und Luft unterschieden werden. Zudem kann das erste Kontrastmittel vom zweiten Kontrastmittel unterschieden werden. Basierend auf den ersten Projektionsmessdaten und den zweiten Projektionsmessdaten können das erste Kontrastmittel und das zweite Kontrastmittel von zwei aus Fettgewebe, Gewebe, dichtes Gewebe, Knochen und Luft unterschieden werden. Vorteilhaft kann die Erkennung von Knochenstrukturen mit geringer Dichte verbessert werden, da einige Algorithmen diese nur sehr begrenzt von Blutgefäßen mit Kontrastmittel unterscheiden können. Durch eine automatische Segmentierung können die Volumenanteile der Basismaterialien für jede Region getrennt ausgegeben werden. Ein Verfahren zur Multimaterialzerlegung ist beispielsweise aus der Druckschrift DE102009017615 A1 bekannt. Vorteilhaft können mittels der Multimaterialzerlegung mehr als zwei Materialien bzw. Materialpaare voneinander getrennt werden, beispielsweise Wasser oder Weichgewebe, das erste Kontrastmittel und das zweite Kontrastmittel. Als Basismaterialien können beispielsweise Wasser, verschiedene Gewebearten, Knochen, erstes Kontrastmittel oder zweites Kontrastmittel gewählt werden.

Die Erfindung betrifft ferner ein Bilderzeugungseinrichtung zum Durchführen des erfindungsgemäßen Verfahrens, aufweisend eine Eingangsschnittstelle zur Erfassung von mittels eines Computertomographiesystems mit Hilfe einer Aufnahme gewonnenen Projektionsmessdaten von einem Untersuchungsbereich eines zu untersuchenden Objekts, eine Rekonstruktionseinheit zum Rekonstruieren eines ersten Bildes auf der Basis der erfassten ersten Projektionsmessdaten und zweiten Projektionsmessdaten, und eine Bilddaten-Schnittstelle zur Ausgabe des ersten Bildes. Vorteilhaft können alle Schritte des erfindungsgemäßen Verfahrens in der Bilderzeugungseinrichtung durchgeführt werden.

Die Erfindung betrifft ferner ein Computertomographiesystem, aufweisend eine Projektionsdaten-Aufnahmeeinheit, umfassend eine Röntgenquelle und eine Detektorvorrichtung zur Aufnahme von Projektionsmessdaten eines Untersuchungsbereichs eines zu untersuchenden Objekts, eine Steuereinrichtung zur Ansteuerung der Projektionsdaten-Aufnahmeeinheit, und eine erfindungsgemäße Bilderzeugungseinrichtung. Vorteilhaft weist das Computertomographiesystem alle Mittel zum Ausführen des Verfahrens auf.

Die Erfindung betrifft ferner ein Computerprogrammprodukt mit einem Computerprogramm, welches direkt in eine Speichereinrichtung einer Steuereinrichtung eines Computertomographiesystems ladbar ist, mit Programmabschnitten, um alle Schritte des erfindungsgemäßen Verfahrens auszuführen, wenn das Computerprogramm in der Steuereinrichtung des Computertomographiesystems ausgeführt wird. Vorteilhaft kann das Computerprogrammprodukt dazu genutzt werden, dass ein Computertomographiesystem das erfindungsgemäße Verfahren ausführen kann.

Die Erfindung betrifft ferner ein computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden. Vorteilhaft kann das computerlesbare Medium dazu genutzt werden, dass ein Computertomographiesystem das erfindungsgemäße Verfahren ausführen kann.

Nachfolgend werden Ausführungsbeispiele der Erfindung anhand von Zeichnungen näher erläutert. Hierbei zeigt:
FIG 1 schematisch ein Konzept eines erfindungsgemäßen Verfahrens gemäß einer ersten Ausführungsform;
FIG 2 schematisch ein Konzept eines erfindungsgemäßen Verfahrens gemäß einer zweiten Ausführungsform;
FIG 3 schematisch ein Konzept eines erfindungsgemäßen Verfahrens gemäß einer dritten Ausführungsform;
FIG 4 schematisch ein Konzept eines erfindungsgemäßen Verfahrens gemäß einer vierten Ausführungsform;
FIG 5 schematisch ein Konzept eines erfindungsgemäßen Computertomographen gemäß einer ersten Ausführungsform; und
FIG 6 schematisch ein Konzept eines erfindungsgemäßen Computertomographen gemäß einer zweiten Ausführungsform.

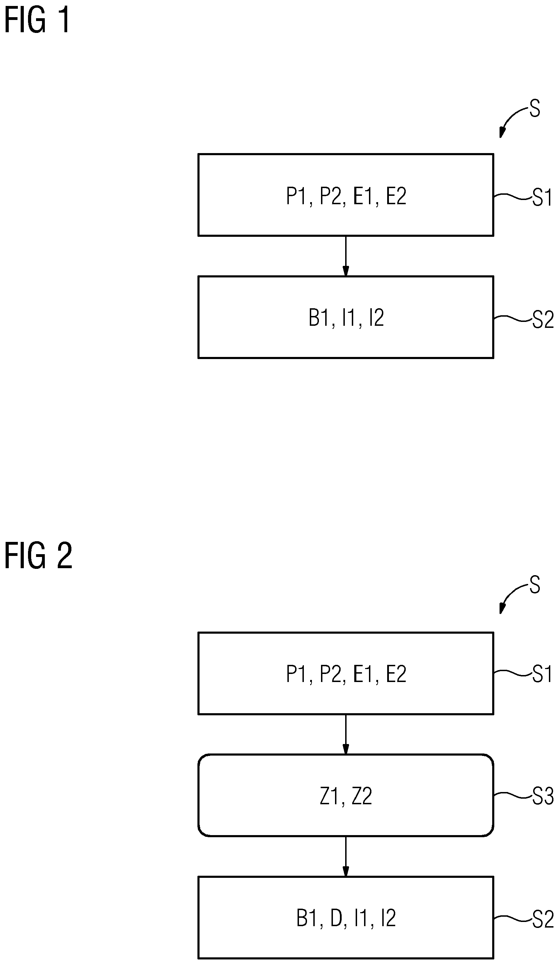

Die Fig. 1 zeigt eine beispielhafte Ausführung des erfindungsgemäßen Verfahrens S zur Bildgebung eines Untersuchungsbereichs eines zu untersuchenden Objekts mit einem Computertomographiesystem gemäß einer ersten Ausführungsform. Der Untersuchungsbereich weist ein erstes Kontrastmittel und ein vom ersten Kontrastmittel verschiedenes zweites Kontrastmittel auf. Das Verfahren S weist zumindest die Schritte der Aufnahme S1 und des Erzeugens S2 auf. Im Schritt der Aufnahme S1 werden erste Projektionsmessdaten P1 mit einem ersten Energiebereich E1 und von den ersten Projektionsmessdaten P1 verschiedenen zweiten Projektionsmessdaten P2 mit einem von dem ersten Energiebereich E1 verschiedenen zweiten Energiebereich E2 in einem Untersuchungsbereich aufgenommen. Der erste Energiebereich E1 umfasst ein erstes Röntgenspektrum und der zweite Energiebereich E2 umfasst ein zweites Röntgenspektrum. Zusätzlich oder alternativ umfasst der erste Energiebereich E1 einen ersten Detektorenergiebereich und der zweite Energiebereich E2 einen zweiten Detektorenergiebereich. Es werden spektrale Projektionsmessdaten P1, P2 aufgenommen, so dass eine spektrale Technik zur Erzeugung S2 des ersten Bildes B1 angewendet werden kann. Im Schritt der Erzeugung S2 wird ein erstes Bild B1 auf Basis der ersten Projektionsmessdaten P1 und der zweiten Projektionsmessdaten P2 erzeugt. Das erste Bild B1 weist ferner nur isolierte erste Informationen I1 des ersten Kontrastmittels, nur isolierte zweite Informationen 12 des zweiten Kontrastmittels, oder isolierte erste Informationen I1 des ersten Kontrastmittels gemeinsam mit isolierten zweiten Informationen 12 des zweiten Kontrastmittels auf.

Das erste Kontrastmittel weist eine therapeutische wirksame Funktionalisierung auf. Die therapeutische wirksame Funktionalisierung umfasst an das erste Kontrastmittel gebundene chemische oder/und radioaktive Substanzen. Das erste Kontrastmittel weist eine an Mikrokugeln oder Nanopartikel gebundene Kontrastsubstanz auf. Das erste Kontrastmittel weist mindestens eines, bevorzugt genau eines, der Elemente Holmium, Eisen, Gold, Wolfram oder Gadolinium auf. Das zweite Kontrastmittel ist für eine Blutflusseigenschaft, beispielsweise eine Durchblutung, indikativ. Das zweite Kontrastmittel weist mindestens eines, bevorzugt genau eines, der Elemente Jod oder Gadolinium auf.

Das erste Bild B1 kann eine lokale Information über eine lokale Verteilung des ersten Kontrastmittels oder/und zweiten Kontrastmittels aufweisen. Das erste Bild B1 kann eine lokale Information über eine Menge des ersten Kontrastmittels oder/und des zweiten Kontrastmittels aufweisen. Das erste Bild B1 kann eine lokale Information über eine Menge oder Verteilung des ersten Kontrastmittels aufweisend eine therapeutische wirksame Funktionalisierung aufweisen. Das erste Bild B1 kann eine lokale Information über eine lokale Verteilung des ersten Kontrastmittels oder/und des zweiten Kontrastmittels in einem vorbestimmten Bereich aufweisen. Das erste Bild B1 kann eine lokale Information basierend auf dem zweiten Kontrastmittel über eine lokale Durchblutung in einem vorbestimmten Bereich aufweisen.

Die Fig. 2 zeigt eine beispielhafte Ausführung des erfindungsgemäßen Verfahrens S zur Bildgebung eines Untersuchungsbereichs eines zu untersuchenden Objekts mit einem Computertomographiesystem gemäß einer zweiten Ausführungsform. Das Verfahren weist ferner einen Schritt der Differenzbildung S3 auf. Das erste Kontrastmittel oder das zweite Kontrastmittel weist ein Element auf, welches eine K-Kante im Bereich von 30 bis 100keV aufweist. Beispielsweise weisen in diesem Bereich Jod, Gadolinium und Gold eine K-Kante auf. Ferner sind Jod, Gadolinium und Gold als Kontrastmittel geeignet. Durch die K-Kante des ersten Kontrastmittels oder des zweiten Kontrastmittels kann mittels spektraler Technik, insbesondere einem zählenden bzw. direkt-konvertierenden Röntgendetektor, der erreichbare Kontrast im beispielsweise ersten Bild oder zweiten Bild vorteilhaft erhöht wird.

Der erste Energiebereich E1 oder der zweite Energiebereich E2 kann bevorzugt an die Photonenenergie, bei der der Energiewert der K-Kante erreicht bzw. überschritten ist, angepasst sein. Es kann zunächst im Schritt der Zwischenbilderzeugung S3 für den ersten Energiebereich E1 ein erstes Zwischenbild Z1 und für den zweiten Energiebereich E2 ein zweites Zwischenbild Z2 erzeugt werden. Anschließend kann ein Differenzbild D aus dem ersten Zwischenbild Z1 und dem zweiten Zwischenbild Z2 erzeugt werden. Dabei kann die unterschiedliche Abhängigkeit der Schwächungskoeffizienten von der Photonenenergie der Röntgenstrahlung des ersten Kontrastmittels und des zweiten Kontrastmittels vorteilhaft genutzt werden, wobei die Abhängigkeit besonders stark im Bereich der K-Kante ausgeprägt ist. Besonders vorteilhaft ist die Wahl des ersten Energiebereichs E1 und des zweiten Energiebereichs E2, wenn jeweils einer der beiden Energiebereiche oberhalb bzw. unterhalb des Energiewerts der K-Kante ausgebildet ist. Bei einem zählenden Röntgendetektor kann eine Energieschwelle bzw. Diskriminatorschwelle zwischen dem ersten Detektorenergiebereich und dem zweiten Detektorenergiebereich an die K-Kante des ersten Kontrastmittels oder des zweiten Kontrastmittels angepasst sein. Beispielsweise kann die Energieschwelle im Wesentlichen der Energie der K-Kante entsprechen. Das Differenzbild D kann das erste Bild B1 oder das zweite Bild B2 sein. Das Differenzbild D kann nur isolierte Informationen des Kontrastmittels, welches ein Element mit einer K-Kante aufweist, aufweisen.

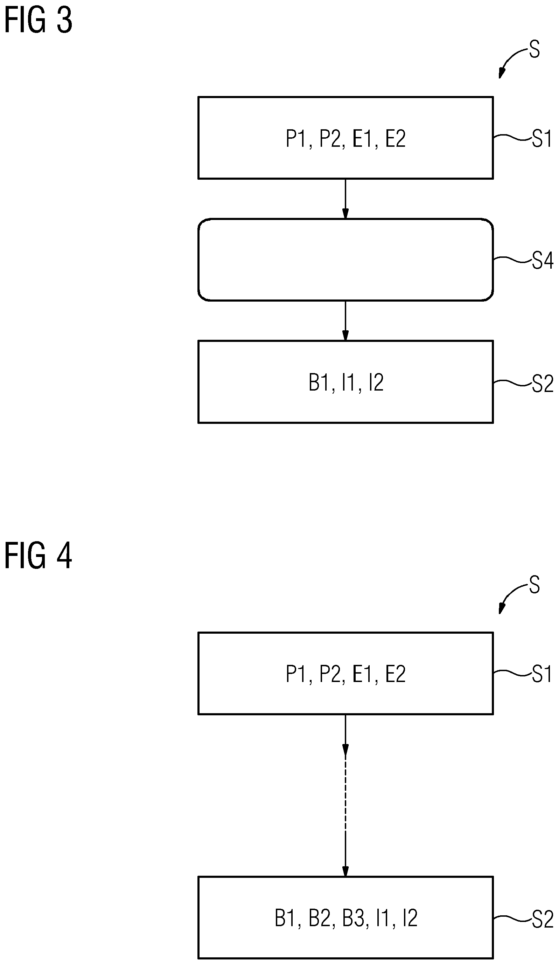

Die Fig. 3 zeigt eine beispielhafte Ausführung des erfindungsgemäßen Verfahrens S zur Bildgebung eines Untersuchungsbereichs eines zu untersuchenden Objekts mit einem Computertomographiesystem gemäß einer dritten Ausführungsform. Das Verfahren S weist ferner einen Schritt der Multimaterialzerlegung S4 auf. Die Multimaterialzerlegung S4 kann rohdatenbasiert oder bilddatenbasiert erfolgen. Ein Verfahren zur Multimaterialzerlegung S4 ist beispielsweise aus der Druckschrift DE102009017615 A1 bekannt. Vorteilhaft können mehr als zwei Materialien bzw. Materialpaare voneinander getrennt werden, beispielsweise Wasser oder Weichgewebe, das erste Kontrastmittel und das zweite Kontrastmittel.

Die Fig. 4 zeigt eine beispielhafte Ausführung des erfindungsgemäßen Verfahrens S zur Bildgebung eines Untersuchungsbereichs eines zu untersuchenden Objekts mit einem Computertomographiesystem gemäß einer vierten Ausführungsform. Die Verfahren S gemäß der ersten, zweiten und dritten Ausführungsform können im Schritt der Erzeugung S2 ferner das zweite Bild B2 oder/und das dritte Bild B3 erzeugen.

Das zweite Bild B2 weist isolierte zweite Informationen 12 des zweiten Kontrastmittels auf, wobei die isolierten ersten Informationen I1 des ersten Kontrastmittels entfernt sind. Alternativ weist das zweite Bild B2 isolierte erste Informationen I1 des ersten Kontrastmittels auf, wobei die isolierten zweiten Informationen 12 des zweiten Kontrastmittels entfernt sind. Im dritten Bild B3 sind die isolierten ersten Informationen I1 des ersten Kontrastmittels und die isolierten zweiten Informationen 12 des zweiten Kontrastmittels entfernt.

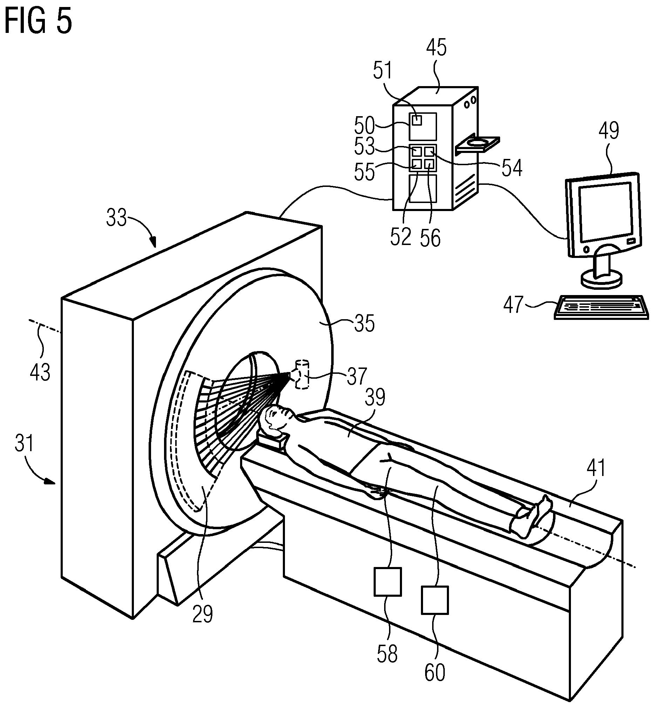

Die Fig. 5 zeigt eine beispielhafte Ausführungsform des erfindungsgemäßen Computertomographiesystems 31 gemäß einer ersten Ausführungsform zum Ausführen des erfindungsgemäßen Verfahrens. Das Computertomographiesystem 31 beinhaltet eine Projektionsdaten-Aufnahmeeinheit 33 mit einem Rotor 35. Der Rotor 35 umfasst eine Röntgenquelle 37 und die Detektorvorrichtung 29. Das Objekt 39 ist auf der Patientenliege 41 gelagert und ist entlang der Rotationsachse z 43 durch die Projektionsdaten-Aufnahmeeinheit 33 bewegbar. Zur Steuerung und Berechnung der Schnittbilder wird eine Rechnereinheit 45 verwendet. Die Rechnereinheit 45 umfasst eine Steuereinrichtung 50 mit einer Speichereinrichtung 51. Die Rechnereinheit 45 umfasst ferner eine Bilderzeugungseinrichtung 52 mit einer Eingangsschnittstelle 53, einer Rekonstruktionseinheit 54, einer Bilddaten-Schnittstelle 55 und einer Materialzerlegungseinheit 56. Die Materialzerlegungseinheit 56 kann zur Durchführung der Multimaterialzerlegung oder/und der Differenzbildung geeignet sein. Eine Eingabeeinrichtung 47 und eine Ausgabevorrichtung 49 sind mit der Rechnereinheit 45 verbunden. Das Computertomographiesystem 31 weist ferner eine erste Injektionseinrichtung 58 zur Injektion des ersten Kontrastmittels auf, wobei die erste Injektionseinrichtung 58 eine Steuereinrichtung zum Ausführen eines patientenspezifischen Injektionsprotokolls umfasst. Das Computertomographiesystem 31 weist ferner eine zweite Injektionseinrichtung 60 zur Injektion des zweiten Kontrastmittels auf, wobei die zweite Injektionseinrichtung 60 eine Steuereinrichtung zum Ausführen eines patientenspezifischen Injektionsprotokolls umfasst. Die Projektionsdaten-Aufnahmeeinheit 33 weist einen direkt-konvertierenden Röntgendetektor in der Detektorvorrichtung 29 bzw. Mittel zum Verändern des Röntgenspektrums bzw. zum Einstellen des ersten Energiebereichs und des zweiten Energiebereichs, beispielsweise mittels Filterung oder kV-Switching, auf.

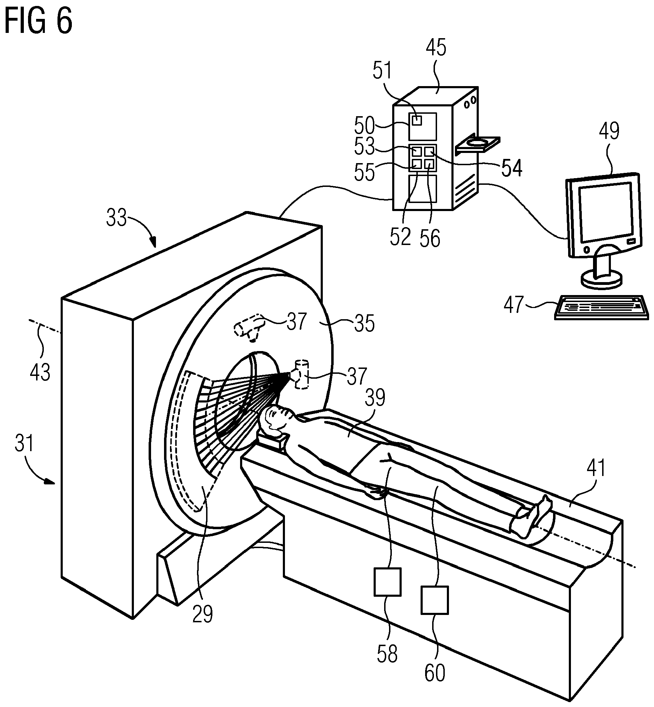

Die Fig. 6 zeigt eine beispielhafte Ausführungsform des erfindungsgemäßen Computertomographiesystems 31 gemäß einer zweiten Ausführungsform zum Ausführen des erfindungsgemäßen Verfahrens. Die Projektionsdaten-Aufnahmeeinheit 33 weist zwei Röntgenquellen 37 und zwei Detektorvorrichtungen 29 (zweite Detektorvorrichtung zur Übersichtlichkeit nicht dargestellt) auf. Das Computertomographiesystem 31 ist ein Dual-Source-CT.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (S) zur Bildgebung eines Untersuchungsbereichs eines zu untersuchenden Objekts (39), wobei der Untersuchungsbereich ein erstes Kontrastmittel und ein vom ersten Kontrastmittel verschiedenes zweites Kontrastmittel aufweist, mit einem Computertomographiesystem (31) aufweisend die Schritte:
a. Aufnahme (S1) von ersten Projektionsmessdaten (P1) mit einem ersten Energiebereich (E1) und von den ersten Projektionsmessdaten (P1) verschiedenen zweiten Projektionsmessdaten (P2) mit einem von dem ersten Energiebereich (E1) verschiedenen zweiten Energiebereich (E2) im Untersuchungsbereich,
b. Erzeugung (S2) eines ersten Bildes (B1) auf Basis der ersten Projektionsmessdaten (P1) und der zweiten Projektionsmessdaten (P2) und ferner aufweisend nur isolierte erste Informationen (I1) des ersten Kontrastmittels, nur isolierte zweite Informationen (12) des zweiten Kontrastmittels, oder isolierte erste Informationen (I1) des ersten Kontrastmittels gemeinsam mit isolierten zweiten Informationen (12) des zweiten Kontrastmittels.

2. Verfahren (S) nach Anspruch 1, wobei das erste Kontrastmittel eine therapeutische wirksame Funktionalisierung aufweist.

3. Verfahren (S) nach einem der vorangehenden Ansprüche, wobei das zweite Kontrastmittel für eine Blutflusseigenschaft indikativ ist.

4. Verfahren (S) nach einem der vorangehenden Ansprüche, wobei das erste Bild (B1) eine lokale Information über eine lokale Verteilung des ersten Kontrastmittels oder/und zweiten Kontrastmittels aufweist.

5. Verfahren (S) nach einem der vorangehenden Ansprüche, wobei das erste Bild (B1) eine lokale Information über eine Menge des ersten Kontrastmittels oder/und des zweiten Kontrastmittels aufweist.

6. Verfahren (S) nach einem der vorangehenden Ansprüche, wobei das erste Bild (B1) eine lokale Information über eine Menge oder Verteilung des ersten Kontrastmittels aufweisend eine therapeutische wirksame Funktionalisierung aufweist.

7. Verfahren (S) nach Anspruch 6, wobei die therapeutische wirksame Funktionalisierung an das erste Kontrastmittel gebundene chemische oder/und radioaktive Substanzen umfasst.

8. Verfahren (S) nach einem der vorangehenden Ansprüche, wobei das erste Bild (B1) eine lokale Information basierend auf dem zweiten Kontrastmittel über eine lokale Durchblutung in einem vorbestimmten Bereich aufweist.

9. Verfahren (S) nach einem der vorangehenden Ansprüche, wobei das erste Kontrastmittel eine an Mikrokugeln oder Nanopartikel gebundene Kontrastsubstanz aufweist.

10. Verfahren (S) nach einem der vorangehenden Ansprüche, wobei der erste Energiebereich (E1) ein erstes Röntgenspektrum und der zweite Energiebereich (E2) ein zweites Röntgenspektrum umfasst.

11. Verfahren (S) nach einem der vorangehenden Ansprüche, wobei der erste Energiebereich (E1) einen ersten Detektorenergiebereich und der zweite Energiebereich (E2) einen zweiten Detektorenergiebereich umfasst.

12. Verfahren (S) nach einem der vorangehenden Ansprüche, wobei ein Schritt der Multimaterialzerlegung (S4) umfasst ist.

13. Bilderzeugungseinrichtung (52) zum Durchführen eines Verfahrens (S) nach einem der Ansprüche 1 bis 12, aufweisend:
a. eine Eingangsschnittstelle (53) zur Erfassung von mittels eines Computertomographiesystems (31) mit Hilfe einer Aufnahme gewonnenen Projektionsmessdaten (P1, P2) von einem Untersuchungsbereich eines zu untersuchenden Objekts (39),
b. eine Rekonstruktionseinheit (54) zum Rekonstruieren eines ersten Bildes (B1) auf der Basis der erfassten Projektionsmessdaten (P1, P2), und
c. eine Bilddaten-Schnittstelle (55) zur Ausgabe des ersten Bildes (B1).

14. Computertomographiesystem (31), aufweisend:
a. eine Projektionsdaten-Aufnahmeeinheit (33), umfassend eine Röntgenquelle (37) und eine Detektorvorrichtung (29) zur Aufnahme von Projektionsmessdaten (P1, P2) eines Untersuchungsbereichs eines zu untersuchenden Objekts (39),
b. eine Steuereinrichtung (50) zur Ansteuerung der Projektionsdaten-Aufnahmeeinheit (33), und
c. eine Bilderzeugungseinrichtung (52) nach Anspruch 13.

15. Computerprogrammprodukt mit einem Computerprogramm, welches direkt in eine Speichereinrichtung (51) einer Steuereinrichtung (50) eines Computertomographiesystems (31) ladbar ist, mit Programmabschnitten, um alle Schritte eines Verfahrens (S) nach einem der Ansprüche 1 bis 12 auszuführen, wenn das Computerprogramm in der Steuereinrichtung (50) des Computertomographiesystems (31) ausgeführt wird.

16. Computerlesbares Medium, auf welchem von einer Rechnereinheit (45) einlesbare und ausführbare Programmabschnitte gespeichert sind, um alle Schritte eines Verfahrens (S) nach einem der Ansprüche 1 bis 12 auszuführen, wenn die Programmabschnitte von der Rechnereinheit (45) ausgeführt werden.
